# EUROPEAN PATENT APPLICATION

(11) **EP 0 974 320 A1**
(43) Date of publication of application: **26.01.2000**
(21) Application number: 98109526.8
(22) Date of filing: 26.05.1998
(51) Int. Cl.: A61F 9/00, A61M 5/178, A61K 9/48

(54) **Medical device for dispensing viscoelastic compositions**

(71) Applicant: Novartis AG, 4058 Basel (CH)
(72) Inventor: The designation of the inventor has not yet been filed

(57) **Abstract**

The present invention relates to a medical device for dispensing viscoelastic compositions characterized in that there are comprised at least two discrete phases of a viscoelastic composition each exhibiting a different viscosity.

## Description

The present invention relates to a medical device for dispensing viscoelastic compositions characterized in that there are comprised at least two discrete phases of a viscoelastic composition each exhibiting a different viscosity.

Cataract surgery typically consists in extracting the crystalline ocular lens that has become opaque. For said surgery several sharp-edged instruments are used in particular in the initial stage of removing said opaque nucleus. It is therefore necessary to protect the corneal endothelium and other surrounding tissues during cataract surgery. A typical example of cataract surgery is disclosed infra. It is generally divided into several stages, namely:
(1) The anterior part of an eye is circularly opened, and usually a highly viscous viscoelastic composition (H+) is injected for the capsulorhexis, which consists of the opening of the capsular bag anteriour face.
(2) Subsequently, manual extraction or phakoemulsification is carried out, which consists typically of an emulsification of the crystalline core with an ultrasound probe related to a special equipment allowing to irrigate and aspirate liquids at the same time.
(3) The capsular bag is then preferably filled with a viscoelastic composition of low viscosity (L-), the intraocular lens (IOL) itself is also covered with viscoelastic composition and is then inserted and positioned.
(4)The viscoelastic composition (L-) is removed and the anterior chamber is closed.

The viscoelastic composition may be administered from a syringe which typically contains a precisely defined volume of sterile viscoelastic composition suitable for cataract surgery. The capsular bag containing the ocular lens and the opened anteriour chamber must be filled with viscoelastic composition in order to maintain the deepness of the internal space, which has a tendency to collapse, which trend is usually significantly increased by nucleus extraction. The volume of the viscoelastic composition may therefore range within a comparable volume since it may temporarely replace the nucleus during surgery. As pointed out above there may be a need to use at least two different grades of viscosity, namely firstly a high viscosity during the extraction of the opaque crystalline lens and secondly a low viscosity for the insertion of the intraocular lens. Sometimes it may be convenient to apply first (L-) and later (H+), depending on the surgeon's own technique and belief, and the type of surgery performed; a viscoelastic composition can be used in several other ocular surgery procedures.

Viscoelastic compositions suitable for cataract surgery are commercially available. Said viscoelastic material is typically contained in a syringe. Furthermore, several viscosity grades of viscoelastic compositions are commercially available too, e.g. high viscosity grade viscoelastic compositions and low viscosity grade viscoelastic compositions. Consequently for an above disclosed cataract surgery, a surgeon would typically need at least two different grades of viscoelastic compositions and consequently, at least two syringes per eye operation. This may generate problems in terms of handling and further in terms of mistakes. Consequently, there exists a need to improve the handling and to avoid potential mistakes e.g. by omitting the plurality of dispensing devices currently needed.

The problem has surprisingly been solved by just charging sequentially a dispensing device with a first viscoelastic composition exhibiting a first defined viscosity, and subsequently with another viscoelastic composition exhibiting another viscosity. After having charged said dispensing device, it is closed with means. These closing means may be moveable and may further be suitable to control the dispensation of the viscoelastic compositions. Said means typically limit the inner space of the present device preferably exactly to the volume of the viscoelastic compositions. Said viscoelastic compositions remain as discrete zones in said device without being mixed in a substantial manner. The viscoelastic compositions in accordance to the present invention may typically be irreversibly miscible if subjected to intense conventional mixing procedure such as mechanical stirring.

When dispensing the viscoelastic compositions, the first zone of viscoelastic composition located at the outlet of the container is dispensed first, e.g. by engaging dispensing means, and subsequently another zone of viscoelastic composition is dispensed by further engaging said dispensing means. Consequently, only one device is needed for the dispensation of at least two different phases of viscoelastic compositions.

The single chamber of the present dispensing device is typically charged with a first viscoelastic composition exhibiting a defined viscosity and a defined composition. Said first composition defines a first phase of viscoelastic composition. Subsequently, said chamber comprising said first phase is typically charged with another viscoelastic composition exhibiting another viscosity and another composition. Said other composition creating another discrete phase above said first phase, will typically build up a separation layer to said first phase. Said charging procedure may be carried on with a further viscoelastic composition exhibiting a further viscosity and a further composition. After having charged said dispensing device, it is closed with closing means. The closing means may typically be moveable and may further be suitable to control the dispensation of said viscoelastic compositions. Said closing means typically limit the inner space of the present device preferably exactly to the volume of the viscoelastic compositions. Said viscoelastic compositions typically remain as discrete zones in said single chamber of the above device without being mixed in a substantial manner.

The term single chamber refers to a container body, vessel, cylinder or the like, which does not contain any physical separating means, and which container body, vessel, cylinder or the like define the dimension of said single chamber.

Preferably the single chamber of the device may be entirely filled with viscoelastic composition such that there remains no free space within said chamber. Consequently, if said single chamber is entirely filled, the viscoelastic composition cannot move freely within said single chamber. Additionally, accumulation of gas, such as air, may not occur by such an arrangement. Therefore, the dispensation of viscoelastic composition is uniform and perfectly controllable.

The device may further contain means for visualizing (indicating means) each discrete zone or phase comprised in the dispensing device. This may be a physiological acceptable dye which selectively tints the viscoelastic composition without tinting the intraocular lens nor the corneal endothelium. Another possibility to visualize the discrete zones of viscoelastic compostions may be indicated by a perforated plastic disk or a plastic ring located between two discrete zones with the proviso that said perforated plastic disk or said plastic ring must not physically separate the viscoelastic compositions.

The invention will be disclosed more particularly in the following, and described by referring to the attached drawing, wherein:
Figure 1 shows schematically a side view of one embodiment of a dispensing device according to the invention;
Figure 2 shows schematically a side view of a further embodiment of a dispensing device according to the invention.

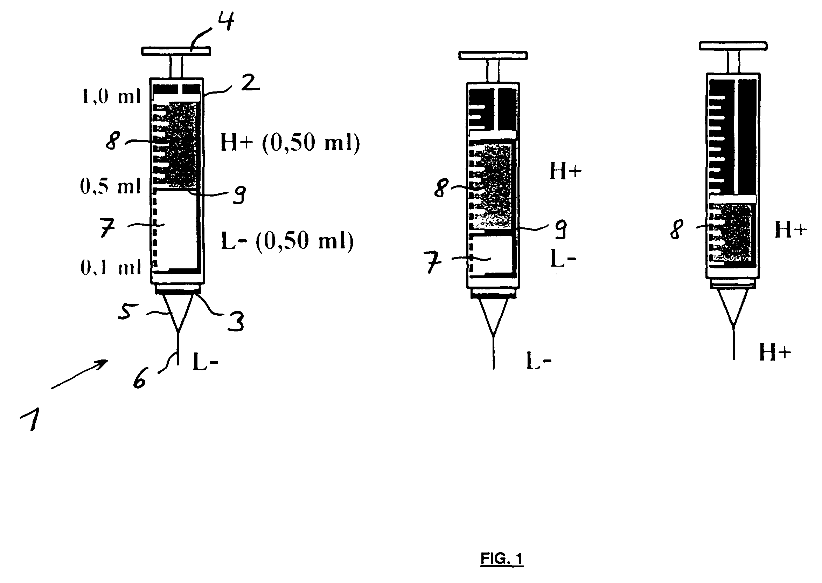

Figure 1 shows a dispensing device in the form of a syringe 1, consisting of a cylindrical body 2, an outlet 3, and a piston 4. The outlet 3 consists of a tip 5 and a cannula 6. The syringe 1 comprises in total a volume of about 1 ml of viscoelastic material, wherein the lower part contains typically about 0.5 ml of low viscosity (L-) viscoelastic material 7. Above said first zone 7, the syringe 1 contains a second layer of typically 0.5 ml of a high viscosity (H+) viscoelastic composition 8. The zone 7 consisting of (L-) viscoelastic composition typically builds up a separation layer 9 to the (H+) viscoelastic composition 8.

Viscoelastic compositions are preferably selected from high molecular weight compounds such as hyaluronic acid, hydroxypropylmethylcellulose, chondroitin sulfate and / or pharmaceutically acceptable salts thereof and / or mixtures thereof, which may be dissolved in a carrier. Other viscoelastic compositions may be selected from acrylates or methacrylates, such as salts of polyacrylic acid or ethyl acrylate, polyacrylamides, natural products, such as gelatin, alginates, pectins, tragacanth, karaya gum, xanthan gum, carrageenin, agar and acacia, starch derivatives, such as starch acetate and hydroxypropyl starch, and also other synthetic products, such as polyvinyl alcohol, polyvinylpyrrolidone, polyvinyl methyl ether, polyethylene oxide, and mixtures of these polymers. A highly preferred viscoelastic composition is hyaluronic acid sodium salt dissolved in water.

A carrier may be selected preferably from water, C₁ - C₇ - alkanols, C₂ - C₆ - glycols, polyethylene glycols, and mixtures thereof and the like. Highly preferred is water.

The difference in the viscosity grade used in accordance to the present invention is typically in the range of from 20% to 10'000%, preferably from 50% to 5'000% and more preferably of from 100% to 1000%.

A viscoelastic composition exhibits typically a viscosity at rest of from about 1'000 cps to about 10'000'000 cps, preferably of from about 10'000 cps to about 8'000'000 cps and more preferably of from about 30'000 cps to about 5'000'000 cps.

The molecular weight of hyaluronic acid may vary of from about 100'000 to about 10'000'000, preferably from about 500'000 to about 5'000'000, more preferably of from about 1'000'000 to about 4'000'000 and most preferably of from about 2'500'000 to about 3'200'000.

The concentration of hyaluronic acid and/or pharmaceutically acceptable salts (e.g. sodium) thereof is typically of from 0.1 % to about 10% by weight, preferably from 0.5% to about 5% by weight and more preferably of from 1.0% to about 3% by weight.

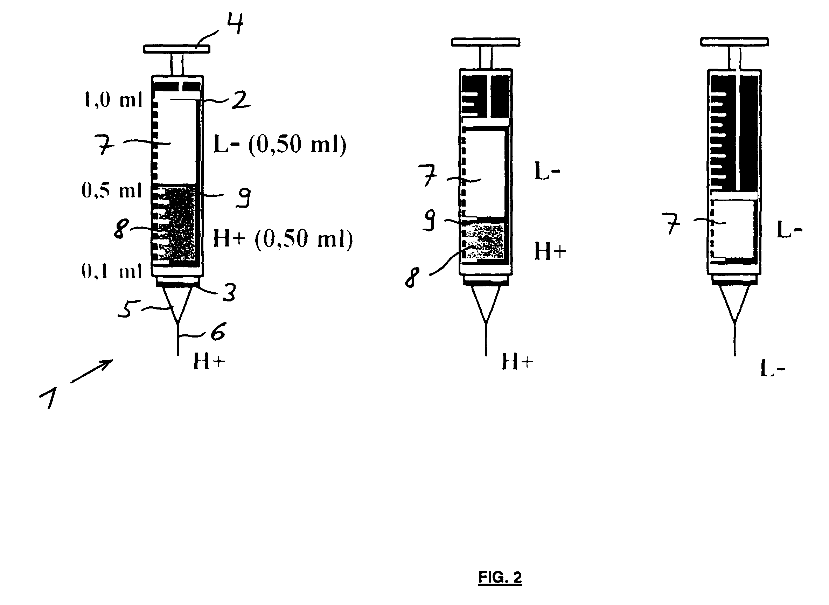

Figure 2 shows a dispensing device in the form of a syringe 1, consisting of the same elements as disclosed in Figure 1, but wherein the zones 7 and 8 have been reversed.

The term discrete phase or discrete zone refers to a defined volume of viscoelastic material exhibiting a constant, defined and stable composition and viscosity.

Within the terms of the present invention the term plastic relates to a polymeric material which is preferably fabricated from polyethylene, polypropylene, Teflon™ and/or mixtures thereof.

The invention further relates to those objects set out in all independent and dependent claims and subclaims disclosed infra.

### Example 1:

A syringe comprising in total 1 ml of viscoelastic material is disclosed (see Figure 1). The lower part contains 0.5 ml of low viscosity (L-) viscoelastic material, which consists of 1 % by weight of sodium hyaluronate (molecular weight = 3'200'000) in water. Above said first zone the device contains a second layer of 0.5 ml of high viscosity (H+) viscoelastic material, which consists of 1.5 % by weight of sodium hyauronate (molecular weight = 2'500'000) in water. The (L-) exhibits a viscosity at rest of about 215'000 cps, the (H+) a viscosity at rest of about 620'000 cps.

The example further discloses the principle of the sequential dispensation of the viscoelastic composition, namely firstly the low viscosity viscoelastic composition (L-), and secondly the high viscosity (H+).
Figure 1 further demonstrates the dispensation of viscoelastic compositions as a function of time. The initial stage of dispensation being disclosed on the left, the final stage of the dispensation being disclosed on the right side of Figure 1. The analogous situation is disclosed in Figure 2 too.

### Example 2:

In contrast to example 1 the reverse situation is disclosed, namely the high viscosity grade viscoelastic composition (H+) is contained in the lower part of the syringe and the (L-) composition in the upper part (see Figure 2).

Consequently, a dispensing device in accordance to the present invention may comprise a random sequence zones each exhibiting different viscosity grades.

As indicated above the present dispensing device is in particular useful in cataract surgery. It has been mentioned too that there is a need to administer at least two different viscoelastic compositions during the various stages of cataract surgery, each exhibiting a different viscosity and a different composition. The viscoelastic compositions comprised in a device in accordance to the invention are injected by the surgeon in a sequential manner. Said viscoelastic compositions do not mix during the dispensation process. Consequently, each viscoelastic composition exhibiting a defined composition and a defined viscosity is injected separately and selectively at will. This process is schematically disclosed in the Figures 1 and 2 (dispensation sequence from left to right). For the dispensation of at least two different viscoelastic compositions, a surgeon may now use one single syringe per eye.

The syringes disclosed in example 1 and 2 are subjected to strong positive and negative accelerations and to vibrations for several weeks. The zones of (H+) and (L-) viscosity grade compositions remained in their original positions without showing any significant signs of being mixed or dislocated. Consequently, the device of the present invention comprising in a single chamber at least two discrete phases of a viscoelastic composition each exhibiting a different viscosity seems to be stable.

For long term storage purposes it is recommended to store the syringes of example 1 and 2 in a vertical position and preferably at a temperature of about 2 to 8°C.

## Claims

1. A device for dispensing viscoelastic compositions, comprising a container body defining a single chamber (2) for retaining viscoelastic compositions, said container having a container outlet (3) defining a longitudinal axis of said single chamber (2), and means (4) for engaging outflow of said viscoelastic compositions through said outlet (3);
wherein said viscoelastic compositions contain at least two discrete phases (7, 8) each exhibiting a different viscosity and a different composition,
wherein said phases (7, 8) are arranged in a sequential manner relative to said longitudinal axis,
wherein each phase (7, 8) builds up a separation layer (9) to another phase (7, 8),
wherein each seperation layer (9) is oriented orthogonal to said longitudinal axis, and wherein said engaging means (4) are operated parallel to the direction of said longitudinal axis.

2. Device of claim 1 which is a medical device.

3. Device of claim 1 wherein the viscoelastic composition is selected from hydroxypropylmethylcellulose, chondroitin sulfate, hyaluronic acid and/or a pharmaceutically acceptable salt thereof and/or mixtures thereof each of which may be dissolved in a pharmaceutically acceptable carrier.

4. Device of claim 1 wherein the carrier is water.

5. Device of claim 1 wherein the viscoelastic composition is sodium hyaluronate in water.

6. Device of claim 1 comprising two phases of sodium hyaluronate in water with different viscosities.

7. Device of claim 1 comprising at least one high viscous viscoelastic composition and at least one low viscous viscoelastic composition.

8. Device of claim 7, wherein the high viscous composition is in the range of from about 300'000 cps to about 5'000'000 cps.

9. Device of claim 7, wherein the low viscous composition is in the range of from about 40'000 cps to about 299'999 cps.

10. Device of claim 1 further comprising means for indicating the separation layer (9) of each discrete phase (7, 8).

11. Device of claim 10 wherein the indicator is a dye which selectively tints the viscoelastic composition without tinting the intraocular lens nor the corneal endothelium.

12. Device of claim 10 wherein the indicator is a perforated plastic disk.

13. Device of claim 2 which is a syringe (1).

14. Device of claim 13 which is a glass or plastic syringe (1).

15. Device of claim 1 wherein the single chamber has a volume in the range of from about 0.1 ml to about 5.0 ml, preferably of from about 0.3 ml to about 3.0 ml and more preferably of from about 0.6 ml to about 2.0 ml.

16. Process for the manufacture of a device in accordance to any of the preceding claims comprising:
(1) providing a single chamber device suitable to carry a viscoelastic composition;
(2) charging said device with a first viscoelastic composition exhibiting a first viscosity;
(3) introducing at least a further amount of viscoelastic composition each exhibiting a different viscosity;
(4) closing said device with means which are preferably suitable to engage the dispensation of the composition.

17. A method of providing in a sequential manner at least two viscoelastic compositions of different viscosities and different compositions to the capsular bag of an ocular lens of a patient in need of cataract surgery, comprising
circular opening of the anterior part of the eye,
injecting a first defined amount of viscoelastic composition exhibiting a defined viscosity and a defined composition through said opening,
removing the ocular lens through said opening by any conventional surgical technique,
wherein said first viscoelastic composition typically protects the endothelium and the surrounding tissues and typically prevents collapse of said capsular bag,
injecting another defined amount of viscoelastic composition exhibiting another defined viscosity and another composition through said opening, wherein said further viscoelastic composition is needed for the insertion of an artificial introcular lens,
wherein the dispensation of said viscoelastic compositions is provided by a single dispensing device.
